# EUROPÄISCHE PATENTANMELDUNG

(11) **EP 1 760 081 A1**
(43) Veröffentlichungstag der Anmeldung: **07.03.2007**
(21) Anmeldenummer: 06022891.3
(22) Anmeldetag: 09.03.2004
(51) Int. Cl.: C07D 413/12, C07D 207/16, C07C 275/26

(54) **Verfahren zur Herstellung von Pyrrolidin-1,2-dicarbonsäure-1-(phenyl(-amid))-2-(phenyl(-amid))Derivaten und 1-(Phenylcarbamoyl)-pyrrolidin-2-carbonsäure Derivaten als Zwischenprodukte**

(30) Priorität: 26.07.2003 DE 10334174
(62) Teilanmeldung aus: 04718646.5
(71) Anmelder: Merck Patent GmbH, 64293 Darmstadt (DE)
(72) Erfinder: Mederski, Werner, 64673, Zwingenberg (DE); Tsaklakidis, Christos, 69469, Weinheim (DE); Dorsch, Dieter, 64372, Ober-Ramstadt (DE); Cezanne, Bertram, 64546, Mörfelden-Walldorf (DE); Gleitz, Johannes, 64289, Darmstadt (DE)

(57) **Zusammenfassung**

Verfahren zur Herstellung von Verbindungen der Formel I 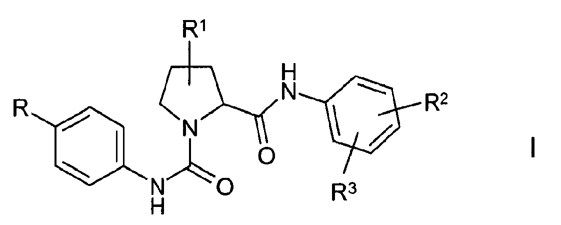 worin R, R¹, R² und R³ die in Patentanspruch 1 angegebene Bedeutung haben,
sowie Verbindungen der Formel IV 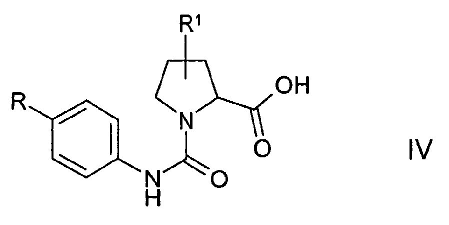 worin
R und R¹ die in Patentanspruch 1 angegebene Bedeutung haben, sind Zwischenprodukte zur Herstellung der Verbindungen der Formel I.

## Beschreibung

Gegenstand der Erfindung ist ein Verfahren zur Herstellung von Verbindungen der Formel I 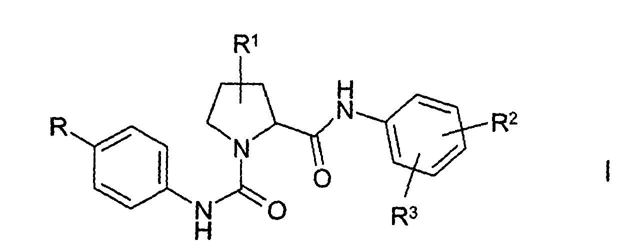 worin
- R: Hal oder C≡CH,
- R¹: H, =O, Hal, A, OH, OA, A-COO-, A-CONH-, A-CONA-, N₃, NH₂, NO₂, CN, COOH, COOA, CONHA, CONH₂, CON(A)₂, O-Allyl, O-Propargyl, O-Benzyl, =N-OH oder =N-OA,
- R²: H, Hal oder A,
- R³: 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H-*Pyrazin-1-yl, 2-Oxo-imidazolidin-1-yl, 2-Imino-piperidin-1-yl, 2-tmino-pyrrolidin-1-yl, 3-lmino-morpholin-4-yl, 2-Imino-imidazolidin-1-yl, 2-Imino-1*H*-Pyrazin-1-yl, 2,6-Dioxo-piperidin-1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl, wobei die Reste auch ein- oder zweimal durch A oder OA substituiert sein können,
- A: unverzweigtes, verzweigtes oder cylisches Alkyl mit 1-10 C-Atomen, worin auch 1-7 H-Atome durch F ersetzt sein können,
- Hal: F, Cl, Br oder I,
bedeuten,
sowie ihrer pharmazeutisch verwendbaren Derivate, Solvate und Stereoisomeren, einschließlich deren Mischungen in allen Verhältnissen, dadurch gekennzeichnet, daß man
a) eine Verbindung der Formel II 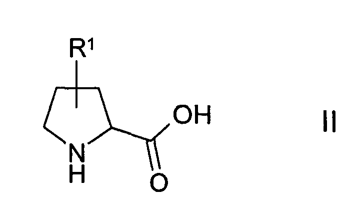 worin
   R¹ die oben angegebenen Bedeutungen hat,
   mit einer Verbindung der Formel III 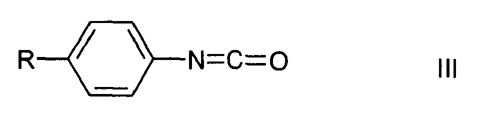 worin
   R die oben angegebenen Bedeutungen hat,
   zu einer Verbindung der Formel IV 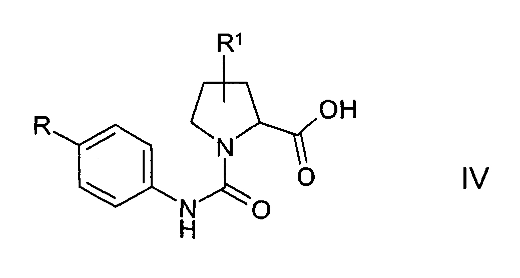 worin
   R und R¹ die oben angegebenen Bedeutungen haben,
   umsetzt,
b) dann eine Verbindung der Formel IV mit einer Verbindung der Formel V 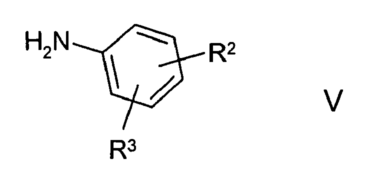 worin R² und R³ die oben angegebenen Bedeutungen haben,
   zu einer Verbindung der Formel I umsetzt, und
c) diese gegebenenfalls in ihre pharmazeutisch verwendbaren Derivate und/oder Solvate überführt,
   indem man eine Base oder Säure der Formel I in eines ihrer Salze umwandelt.

Der Erfindung lag die Aufgabe zugrunde, neue verbesserte Verfahren zur Herstellung von Faktor Xa-Inhibitoren aufzufinden.
Im Vergleich zu bekannten Verfahren aus dem Stand der Technik ist das erfindungsgemäße Verfahren kürzer und effizienter.

Faktor Xa Inhibitoren können zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.
Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in Circulation 1996, 94, 1705-1712 erfolgen.

Eine Inhibierung des Faktors Xa kann somit verhindern, daß Thrombin gebildet wird.
Die Inhibierung des Faktors Xa und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in Thrombosis and Haemostasis 1990, 63, 220-223 beschrieben.

Die Messung der Inhibierung von Faktor Xa kann z.B. nach der Methode von T. Hara et al. in Thromb. Haemostas. 1994, 71, 314-319 erfolgen.

Der Gerinnungsfaktor Vlla initiiert nach Bindung an Tissue Faktor den extrinsischen Teil der Gerinnungskaskade und trägt zur Aktivierung des Faktors X zu Faktor Xa bei. Eine Inhibierung von Faktor Vlla verhindert somit die Entstehung des Faktors Xa und damit eine nachfolgende Thrombinbildung.
Die Inhibierung des Faktors Vlla und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIa wird z.B. von H. F. Ronning et al. in *Thrombosis Research* **1996**, *84*, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa und die Messung der antikoagulierenden und antithrombotischen Aktivität kann nach üblichen in vitro- oder in vivo-Methoden ermittelt werden. Ein geeignetes Verfahren wird z.B. von J. Chang et al. in Journal of Biological Chemistry 1998, 273, 12089-12094 beschrieben.

Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt. Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

In WO 03/045912 wird ein um 2 Stufen längerer Weg beschrieben, der über ein N-geschütztes Pyrrolidinderivat, z.B. BOC-Pro, führt:

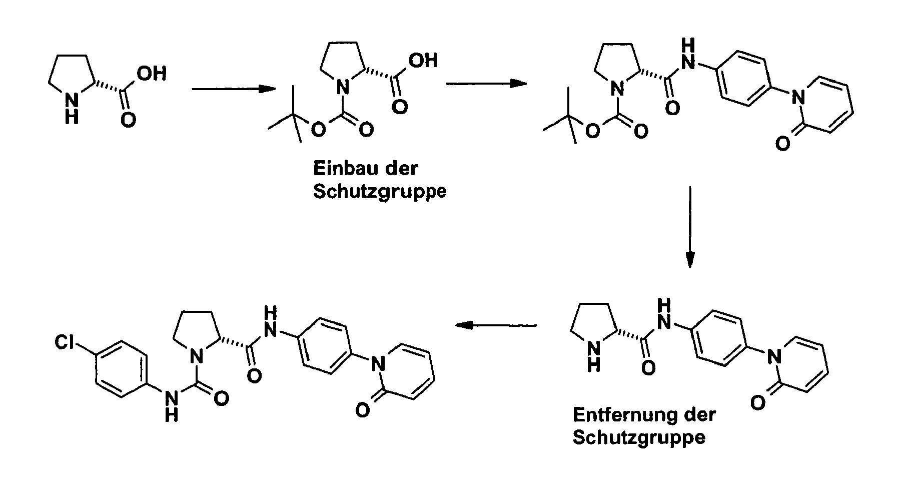

In Helv. Chim. Acta 1998, 81, 1254-1263 wird die Umsetzung von primären Aminen mit 4-Chlorphenylisocyanat beschrieben (Weg A]).
Wie in Weg B] aufgezeigt, reagieren dort auch die reaktiven Seitenkettengruppen, wie OH, NH oder SH zu Bisadditionsprodukten ab.

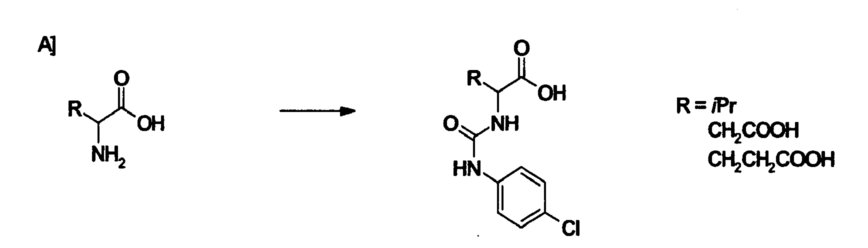

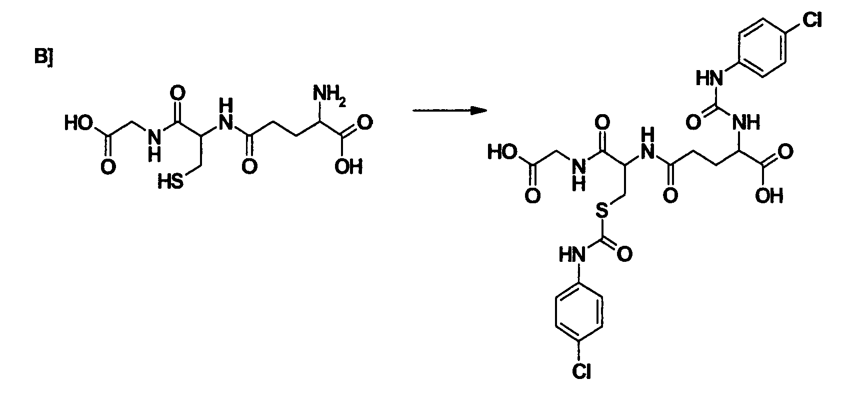

Unter pharmazeutisch verwendbaren Derivaten versteht man z.B. die Salze der Verbindungen als auch sogenannte Prodrug-Verbindungen. Vor- und nachstehend bedeutet A Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl oder Trifluormethyl.

Hal bedeutet vorzugsweise F, Cl oder Br, aber auch I.

Gegenstand der Erfindung ist vorzugsweise ein Verfahren nach Anspruch 1, zur Herstellung von Verbindungen der Formel I, worin R F oder Cl bedeutet.

Bevorzugt ist weiterhin ein Verfahren nach Anspruch 1 oder 2, zur Herstellung von Verbindungen der Formel I, worin
- R¹: H, =O, OH, OA, A-COO-, N₃, NH₂, O-Allyl oder O-Propargyl bedeutet.
Besonders bevorzugt ist ein Verfahren nach Anspruch 1 oder 2, zur Herstellung von Verbindungen der Formel I, worin R¹ H oder OH bedeutet.

Weiter bevorzugt ist ein Verfahren nach Anspruch 1-4, zur Herstellung von Verbindungen der Formel I, worin
- R³: 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H-*pyridazin-2-yl bedeutet.

Weiter bevorzugt ist ein Verfahren nach Anspruch 1-5, zur Herstellung von Verbindungen der Formel I, worin
A unverzweigtes oder verzweigtes Alkyl mit 1-6 C-Atomen, worin auch 1-3 H-Atome durch F ersetzt sein können, bedeutet.

Weiter ist bevorzugt ein Verfahren nach einem oder mehreren der Ansprüche 1-6, zur Herstellung von Verbindungen der Formel I, worin
- R: Hal oder C≡CH,
- R¹: H, OH oder OA,
- R²: H, Hal oder A,
- R³: 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl,
- A: unverzweigtes, verzweigtes oder cylisches Alkyl mit 1-10 C-Atomen, worin auch 1-7 H-Atome durch F ersetzt sein können,
- Hal: F, Cl, Br oder I,
bedeuten,
sowie ihrer pharmazeutisch verwendbaren Derivate, Solvate und Stereoisomeren, einschließlich deren Mischungen in allen Verhältnissen.

Weiter ist bevorzugt ein Verfahren nach einem oder mehreren der Ansprüche 1-7, zur Herstellung von Verbindungen der Formel I, worin
- R: F oder Cl,
- R¹: H, =O, OH, OA, A-COO-, N₃, NH₂, O-Allyl oder O-Propargyl,
- R²: H, F oder A,
- R³: 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H-*Pyrazin-1-yl, 2-Oxo-imidazolidin-1-yl, 2-Oxo-piperazin-1-yl oder 3-Oxo-2*H*-pyridazin-2-yl,
- A: unverzweigtes oder verzweigtes Alkyl mit 1-6 C-Atomen, worin auch 1-3 H-Atome durch F ersetzt sein können,
bedeuten,
sowie ihrer pharmazeutisch verwendbaren Derivate, Solvate und Stereoisomeren, einschließlich deren Mischungen in allen Verhältnissen.

Besonders bevorzugt ist ein Verfahren nach einem oder mehreren der Ansprüche 1-8, zur Herstellung von Verbindungen der Formel I, worin
- R: F oder CI,
- R¹: H oder OH,
- R²: H, F oder A,
- R³: 3-Oxo-morpholin-4-yl,
- A: unverzweigtes oder verzweigtes Alkyl mit 1-6 C-Atomen, worin auch 1-3 H-Atome durch F ersetzt sein können,
bedeuten,
sowie ihrer pharmazeutisch verwendbaren Derivate, Solvate und Stereoisomeren, einschließlich deren Mischungen in allen Verhältnissen.

Ganz besonders bevorzugt ist ein Verfahren nach einem oder mehreren der Ansprüche 1-15, zur Herstellung von Verbindungen der Formel la 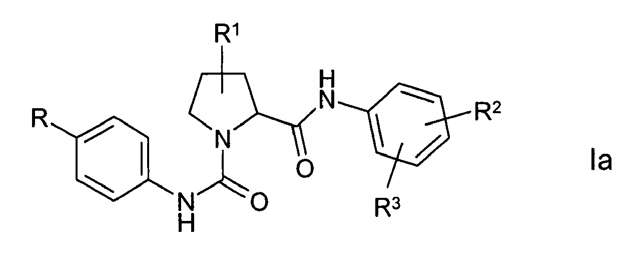 worin
- R: F oder Cl,
- R¹: H oder OH,
- R²: H, F oder A,
- R³: 3-Oxo-morpholin-4-yl,
- A: unverzweigtes oder verzweigtes Alkyl mit 1-6 C-Atomen, worin auch 1-3 H-Atome durch F ersetzt sein können,
bedeuten,
sowie ihrer pharmazeutisch verwendbaren Derivate, Solvate und Stereoisomeren, einschließlich deren Mischungen in allen Verhältnissen, dadurch gekennzeichnet, daß man
a) eine Verbindung der Formel II 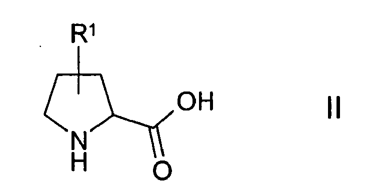 worin
   - R¹: H oder OH bedeutet,
   mit einer Verbindung der Formel III 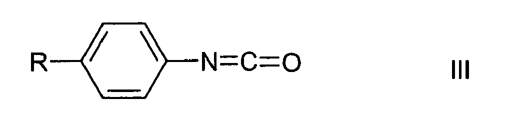 worin
   - R: F oder CI bedeutet,
   in wässriger Alkali- oder Erdalkalimetall-carbonat- oder -bicarbonat-Lösung, bei einer Temperatur zwischen 60° und 110° C,
   zu einer Verbindung der Formel IV 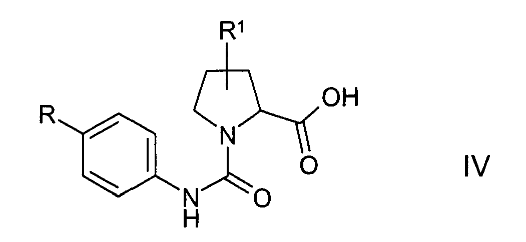 worin
   - R: F oder Cl,
   - R¹: H oder OH
   bedeuten,
   umsetzt,
b) dann eine Verbindung der Formel IV mit einer Verbindung der Formel V 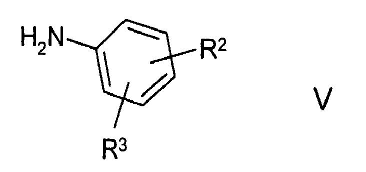 worin
   - R²: H, F oder A,
   - R³: 3-Oxo-morpholin-4-yl,
   - A: unverzweigtes oder verzweigtes Alkyl mit 1-6 C-Atomen, worin auch 1-3 H-Atome durch F ersetzt sein können,
   bedeuten,
   in Gegenwart eines Hilfsreagenzes unter Bildung eines gemischten Anhydrids, bei einer Temperatur zwischen 10° und 70° C,
   zu einer Verbindung der Formel la umsetzt, und
c) diese gegebenenfalls in ihre pharmazeutisch verwendbaren Derivate und/oder Solvate überführt,
   indem man eine Base oder Säure der Formel la in eines ihrer Salze umwandelt.

Die Verbindungen der Formel I bzw. la können vorzugsweise erhalten werden, indem man in einem ersten Schritt a) Verbindungen der Formel II mit Verbindungen der Formel III umsetzt.
Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums, wie z.B. NaOH, Natriumcarbonat, Kaliumcarbonat, Cäsiumcarbonat oder NaHCO₃. Besonders bevorzugt ist NaHCO₃.
Auch der Zusatz einer organischen Base wie Triethylamin, Dimethylanilin, Pyridin oder Chinolin kann günstig sein. Die Reaktionszeit liegt je nach den angewendeten Bedingungen zwischen einigen Minuten und 14 Tagen, vorzugsweise zwischen einer und zehn Stunden, die Reaktionstemperatur zwischen etwa 0° und 150°, normalerweise zwischen 20° und 130°, vorzugsweise zwischen 60° und 110°, ganz besonders bevorzugt zwischen 70° und 90° C.

Als inerte Lösungsmittel eignen sich z.B. Wasser; Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton, Isobutylmethylketon (IBMK) oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt ist Wasser.

In einem zweiten Schritt werden Verbindungen der Formel IV mit Verbindungen der Formel V umgesetzt.
Vorzugsweise erfolgt die Umsetzung in Gegenwart eines Hilfsreagenzes, das mit der OH-Gruppe der Carbonsäure ein intermediäres Derivat bildet, wie z.B. ein gemischtes Anhydrid, einen aktivierten Ester, ein Imidazolid oder Umsetzung zu einer Alkylsulfonyloxy-Gruppe mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy). Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Die Kupplung kann mit unterschiedlichen Kondensationsreagenzien wie Carbodiimiden, Carbodiimidazol, solchen des Uronium-Typs wie TBTU, sowie Säurehalogenid- oder Aktivester-Methoden durchgeführt werden. Aktivierte Ester werden zweckmäßig in situ gebildet, z. B. durch Zusatz von HOBt oder N-Hydroxysuccinimid.

Bevorzugt ist die Bildung eines gemischten Anhydrids.

Besonders bevorzugt ist hier die Verwendung von Ethyl-2-ethoxy-1,2-dihydrochinolin-1-carboxylat (EEDQ).

Die Umsetzung erfolgt in der Regel in einem inerten Lösungsmittel.

Die Reaktionszeit liegt je nach den angewendeten Bedingungen zwischen einigen Minuten und 14 Tagen, vorzugsweise zwischen einer und zwanzig Stunden, die Reaktionstemperatur zwischen etwa 0° und 150°, normalerweise zwischen 0° und 90°, vorzugsweise zwischen 10° und 70°, besonders bevorzugt zwischen 15° und 30° C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel, besonders bevorzugt ist Tetrahydrofuran.

Eine Base der Formel I, la bzw. der Formel IV kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I, la bzw. IV mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Die Erfindung betrifft weiterhin Verbindungen der Formel IV 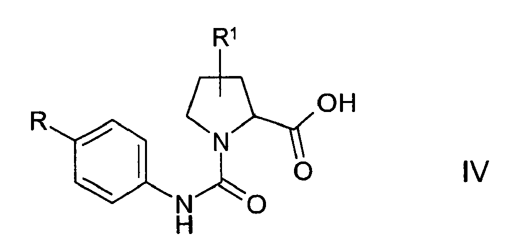 worin
- R: Hal oder C≡CH,
- R¹: H, =O, Hal, A, OH, OA, A-COO-, A-CONH-, A-CONA-, N₃, NH₂, NO₂, CN, COOH, COOA, CONH₂, CONHA, CON(A)₂, O-Allyl, O-Propargyl, O-Benzyl, =N-OH oder =N-OA,
- A: unverzweigtes, verzweigtes oder cylisches Alkyl mit 1-10 C-Atomen, worin auch 1-7 H-Atome durch F ersetzt sein können,
- Hal: F, Cl, Br oder I,
bedeuten,
sowie ihre pharmazeutisch verwendbaren Derivate, Solvate und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate der erfindungsgemäßen Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel IV, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Insbesondere können die Verbindungen der Formel IV in Verfahren zur Herstellung von Verbindungen der Formel I verwendet werden.

Andere Ethinylderivate sind als Faktor Xa-Inhibitoren in der WO 02/079145 beschrieben.
Andere aromatische Amide sind in der WO 99/00121 und in der WO 00/39118 beschrieben. Aromatische Amidinderivate mit antithrombotischer Wirkung sind z.B. aus der EP 0 540 051 B1 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)phenylalkyl]-azaheterocyclylamide als Faktor Xa Inhibitoren sind in WO 96/40679 beschrieben.

Für alle Reste, die mehrfach auftreten, gilt, daß deren Bedeutungen unabhängig voneinander sind.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl oder Trifluormethyl.

Hal bedeutet vorzugsweise F, Cl oder Br, aber auch I.
R bedeutet vorzugsweise F oder Cl,
R¹ bedeutet vorzugsweise H, =O, OH, OA, A-COO-, N₃, NH₂, O-Allyl oder O-Propargyl, besonders bevorzugt H oder OH.

Besonders bevorzugt sind Verbindungen der Formel IV ausgewählt aus der Gruppe
(2R,4R)-1-(4-Chlor-phenylcarbampyl)-4-hydroxy-pyrrolidin-2-carbonsäure,
(2R)-1-(4-Chlor-phenylcarbamoyl)-pyrrolidin-2-carbonsäure,

Erfindungsgemäße Verbindungen der Formel IV können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der Endprodukte, die aus den Zwischenverbindungen resultieren, unterscheiden kann, kann es wünschenswert sein, die Enantiomere der erfindungsgemäßen Verbindungen zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktive Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): EI (Elektronenstoß-lonisation) M⁺; ESI (E-lectrospray lonization) (M+H)⁺; FAB (Fast Atom Bombardment) (M+H)⁺

### Beispiel 1

Die Herstellung von (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} erfolgt analog nachstehendem Schema 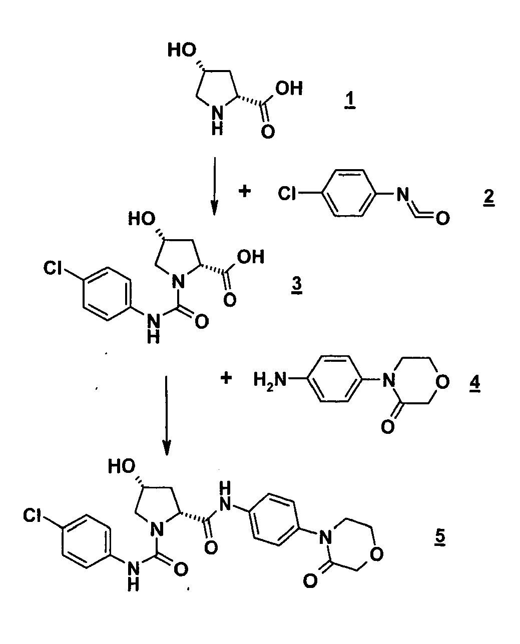
1.1 13,1 g (0,1 mol) Cis-Hydroxy-D-Prolin werden in 800 mL NaHCO₃-Lösung (c = 0,5 mol/L) gelöst, anschließend 30,7 g (0,2 mol) 4-Chlorphenylisocyanat hinzugefügt und 5 h bei 80°C gerührt. Das Reaktionsgemisch wird auf RT abgekühlt, vom ausgefallenen symmetrischen Harnstoff 1,3-Bis(4-chlorphenyl)urethan abgesaugt, mit Wasser gewaschen und die wässrige Phase auf pH = 1 mit ca. 40 mL konz. HCl eingestellt. Das ausgefallene Produkt wird abgetrennt, die wässrige Phase mit EE nachextrahiert und beide organischen Teile getrocknet. Der Rückstand wird nun aus MTB-Ether umkristallisiert. So erhält man 23,3 g (81,8 %) (2R,4R)-1-(4-Chlor-phenylcarbampyl)-4-hydroxy-pyrrolidin-2-carbonsäure 3; F. 132-134°; MS (FAB): m/z = 285 (M+H⁺).
   ¹H NMR (DMSO-d₆) δ 12.00 (sbr, 1 H), 8.39 (s, 1 H), 7.54 (d, *J* = 8.9 Hz, 2H), 7.26 (d, J = 8.9 Hz, 2H), 4.41-4.24 (m, 2H), 3.66 (dd, J = 5.7 and 5.8 Hz, 1 H), 3.33 (dd, J = 4.0 and 4.1 Hz, 1 H), 2.40-2.25 (m, 1 H), 1.96-1.81 (m, 1 H).
   Drehwert: [α]²⁰_{D} = + 43.7°; MeOH, c = 0.0198 g/2mL
   C,H,N: Theorie C 50.63, H 4.60, N 9.84 Gefunden C 51.1 H 4.6 N 9.0
1.2 14,24 g (0,05 mol) 3, 9,61 g (0,05 mol) Aminophenylmorpholinon **4** und 12,37 g (0,05 mol) Ethyl-2-ethoxy-1,2-dihydrochinolin-1-carboxylat werden bei RT in 400 mL Tetrahydrofuran gelöst und 20 h gerührt, wobei sich eine Suspension bildet. Der Niederschlag wird abgesaugt, drei mal mit THF nachgewaschen und im Vakkuum zur Trockne gebracht. Dabei erhält man 15,9 g (69%) (2R,4R)-4-Hydroxy-pyrrolidin-1,2-dicarbonsäure-1-[(4-chlor-phenyl)-amid]-2-{[4-(3-oxo-morpholin-4-yl)-phenyl]-amid} 5;
   F. 208-210°; MS (FAB): m/z = 459 (M+H⁺).

### Analog Beispiel 1.1 erhält man

(2R)-1-(4-Chlor-phenylcarbamoyl)-pyrrolidin-2-carbonsäure, F. 173-175°, 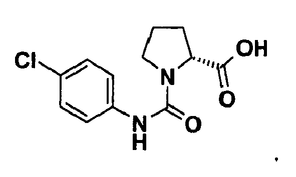 MS (FAB): m/z = 269 (M+H⁺);
¹H NMR (DMSO-d₆) δ 12.37 (sbr, 1 H), 8.37 (s, 1 H), 7.53 (d, *J* = 8.9 Hz, 2H), 7.26 (d, J = 8.9 Hz, 2H), 4.32 (dd, J = 3.5 Hz, 1 H), 3.60-3.41 (m, 2H), 2.27-2.07 (m, 1 H), 2.00-1.81 (m, 3H).
Drehwert: [α]²⁰_{D} = + 60.9°; MeOH, c = 0.0189 g/2mL
C,H,N: Theorie C 53.64, H 4.88, N 10.43 Gefunden C 53.6 H 5.1 N 10.4

### Beispiel 2

Analog Beispiel 1 erhält man die nachstehenden Verbindungen
(2R)-Pyrrolidin-1,2-dicarbonsäure-1-[(4-chlor-phenyl)-amid]-2-{[4-(3-oxo-morpholin-4-yl)-phenyl]-amid},
(2R)-Pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[3-methyl-4-(3-oxo-morpholin-4-yl)-phenyl]-amid},
(2S,4R)-4-Hydroxy-pyrrolidin-1,2-dicarbonsäure-1-[(4-chlor-phenyl)-amid]-2-{[4-(3-oxo-morpholin-4-yl)-phenyl]-amid},
(2R)-Pyrrolidin-1,2-dicarbonsäure-1-[(4-chlor-phenyl)-amid]-2-{[2-fluor-4-(3-oxo-morpholin-4-yl)-phenyl]-amid},
(2R,4S)-4-Hydroxy-pyrrolidin-1,2-dicarbonsäure-1-[(4-chlor-phenyl)-amid]-2-{[4-(3-oxo-morpholin-4-yl)-phenyl]-amid},
(2R)-Pyrrolidin-1,2-dicarbonsäure-1-[(4-chlor-phenyl)-amid]-2-{[3-fluor-4-(3-oxo-morpholin-4-yl)-phenyl]-amid},
(2R)-Pyrrolidin-1,2-dicarbonsäure-1-[(4-chlor-phenyl)-amid]-2-{[3-trifluormethyl-4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[2-fluor-4-(3-oxo-morpholin-4-yl)-phenyl]-amid},
(2R,4S)-4-Azido-pyrrolidin-1,2-dicarbonsäure-1-[(4-chlor-phenyl)-amid]-2-{[4-(3-oxo-morpholin-4-yl)-phenyl]-amid},
(2R,4S)-4-Amino-pyrrolidin-1,2-dicarbonsäure-1-[(4-chlor-phenyl)-amid]-2-{[4-(3-oxo-morpholin-4-yl)-phenyl]-amid},
(2R,4R)-4-Methoxy-pyrrolidin-1,2-dicarbonsäure-1-[(4-chlor-phenyl)-amid]-2-{[4-(3-oxo-morpholin-4-yl)-phenyl]-amid},
(2R,4R)-4-Acetoxy-pyrrolidin-1,2-dicarbonsäure-1-[(4-chlor-phenyl)-amid]-2-{[4-(3-oxo-morpholin-4-yl)-phenyl]-amid},
(2R)-4-Oxo-pyrrolidin-1,2-dicarbonsäure-1-[(4-chlor-phenyl)-amid]-2-{[4-(3-oxo-morpholin-4-yl)-phenyl]-amid},
(2S)-Pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-chlor-phenyl)-amid]-2-{[3-fluor-4-(3-oxo-morpholin-4-yl)-phenyl]-amid},
(2S,4S)-4-Hydroxy-pyrrolidin-1,2-dicarbonsäure-1-[(4-chlor-phenyl)-amid]-2-{[4-(3-oxo-morpholin-4-yl)-phenyl]-amid},
(2R,4R)-4-Allyloxy-pyrrolidin-1,2-dicarbonsäure-1-[(4-chlor-phenyl)-amid]-2-{[4-(3-oxo-morpholin-4-yl)-phenyl]-amid},
(2R,4R)-4-(Prop-2-in-yloxy)-pyrrolidin-1,2-dicarbonsäure-1-[(4-chlor-phenyl)-amid]-2-{[4-(3-oxo-morpholin-4-yl)-phenyl]-amid}.

### Beispiel 3

Die Umsetzung von L-Hydroxyprolin mit 4-Chlorphenylisocyanat kann analog Beispiel 1.1 vorzugsweise auch mit einem Äquivalent Chlorphenylisocyanat vorzugsweise in Isobutylmethylketon (IBMK) durchgeführt werden.

9,4 g (71,685 mmol) L-Hydroxy-prolin werden bei -2 bis 0°C in 71,68 mL NaOH-Lösung (c = 1 mol/L) gelöst, anschließend eine Lösung von 11,008 g (71,685 mmol) 4-Chlorphenylisocyanat in 70 ml IBMK hinzugefügt und 1 h bei -1°C gerührt.
Nach üblicher Aufarbeitung erhält man 18,52 g (2S,4R)-1-(4-Chlorphenylcarbamoyl)-4-hydroxy-pyrrolidin-2-carbonsäure;
Ausbeute: 91 %.

## Patentansprüche

1. Verfahren zur Herstellung von Verbindungen der Formel I 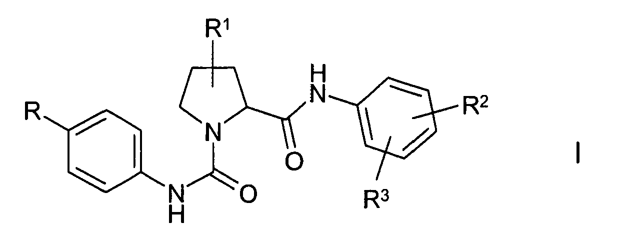 worin
R Hal oder C≡CH,
R¹ H, =O, Hal, A, OH, OA, A-COO-, A-CONH-, A-CONA-, N₃, NH₂, NO₂, CN, COOH, COOA, CONH₂, CONHA, CON(A)₂, O-Allyl, O-Propargyl, O-Benzyl, =N-OH oder =N-OA,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2,6-Dioxo-piperidin-1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl, wobei die Reste auch ein- oder zweimal durch A oder OA substituiert sein können,
A unverzweigtes, verzweigtes oder cylisches Alkyl mit 1-10 C-Atomen, worin auch 1-7 H-Atome durch F ersetzt sein können,
Hal F, Cl, Br oder I,
bedeuten,
sowie ihrer pharmazeutisch verwendbaren Derivate, Solvate und Stereoisomeren, einschließlich deren Mischungen in allen Verhältnissen, **dadurch gekennzeichnet, daß** man
a) eine Verbindung der Formel II 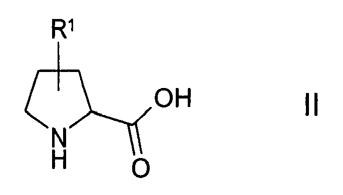 worin
R¹ die oben angegebenen Bedeutungen hat,
mit einer Verbindung der Formel III 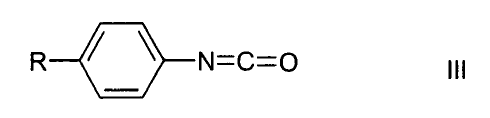 worin
R die oben angegebenen Bedeutungen hat,
zu einer Verbindung der Formel IV 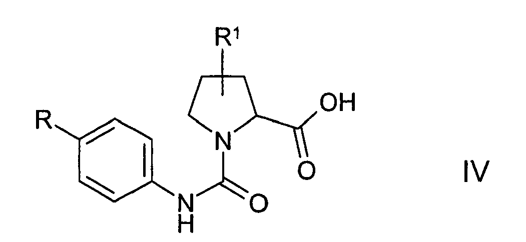 worin
R und R¹ die oben angegebenen Bedeutungen haben,
umsetzt,
b) dann eine Verbindung der Formel IV mit einer Verbindung der Formel V 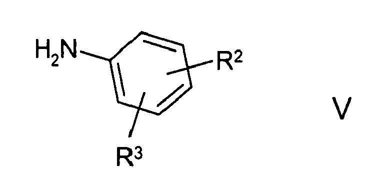 worin R² und R³ die oben angegebenen Bedeutungen haben,
zu einer Verbindung der Formel I umsetzt, und
c) diese gegebenenfalls in ihre pharmazeutisch verwendbaren Derivate und/oder Solvate überführt,
indem man eine Base oder Säure der Formel I in eines ihrer Salze umwandelt.

2. Verfahren nach Anspruch 1, zur Herstellung von Verbindungen der Formel I, worin
R F oder Cl bedeutet,
sowie ihrer pharmazeutisch verwendbaren Derivate, Solvate und Stereoisomeren, einschließlich deren Mischungen in allen Verhältnissen.

3. Verfahren nach Anspruch 1 oder 2, zur Herstellung von Verbindungen der Formel I, worin
R¹ H, =O, OH, OA, A-COO-, N₃, NH₂, O-Allyl oder O-Propargyl bedeutet,
sowie ihrer pharmazeutisch verwendbaren Derivate, Solvate und Stereoisomeren, einschließlich deren Mischungen in allen Verhältnissen.

4. Verfahren nach Anspruch 1, 2 oder 3, zur Herstellung von Verbindungen der Formel I, worin
R¹ H oder OH bedeutet,
sowie ihrer pharmazeutisch verwendbaren Derivate, Solvate und Stereoisomeren, einschließlich deren Mischungen in allen Verhältnissen.

5. Verfahren nach einem oder mehreren der Ansprüche 1-4, zur Herstellung von Verbindungen der Formel I, worin
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl,
bedeutet,
sowie ihrer pharmazeutisch verwendbaren Derivate, Solvate und Stereoisomeren, einschließlich deren Mischungen in allen Verhältnissen.

6. Verfahren nach einem oder mehreren der Ansprüche 1-5, zur Herstellung von Verbindungen der Formel I, worin
A unverzweigtes oder verzweigtes Alkyl mit 1-6 C-Atomen, worin auch 1-3 H-Atome durch F ersetzt sein können,
bedeutet,
sowie ihrer pharmazeutisch verwendbaren Derivate, Solvate und Stereoisomeren, einschließlich deren Mischungen in allen Verhältnissen.

7. Verfahren nach einem oder mehreren der Ansprüche 1-6, zur Herstellung von Verbindungen der Formel I, worin
R Hal oder C≡CH,
R¹ H, OH oder OA,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl,
A unverzweigtes, verzweigtes oder cylisches Alkyl mit 1-10 C-Atomen, worin auch 1-7 H-Atome durch F ersetzt sein können,
Hal F, Cl, Br oder I,
bedeuten,
sowie ihrer pharmazeutisch verwendbaren Derivate, Solvate und Stereoisomeren, einschließlich deren Mischungen in allen Verhältnissen.

8. Verfahren nach einem oder mehreren der Ansprüche 1-7, zur Herstellung von Verbindungen der Formel I, worin
R F oder Cl,
R¹ H, =O, OH, OA, A-COO-, N₃, NH₂, O-Allyl oder O-Propargyl,
R² H, F oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl,
A unverzweigtes oder verzweigtes Alkyl mit 1-6 C-Atomen, worin auch 1-3 H-Atome durch F ersetzt sein können,
bedeuten,
sowie ihrer pharmazeutisch verwendbaren Derivate, Solvate und Stereoisomeren, einschließlich deren Mischungen in allen Verhältnissen.

9. Verfahren nach einem oder mehreren der Ansprüche 1-8, zur Herstellung von Verbindungen der Formel I, worin
R F oder Cl,
R¹ H oder OH,
R² H, F oder A,
R³ 3-Oxo-morpholin-4-yl,
A unverzweigtes oder verzweigtes Alkyl mit 1-6 C-Atomen, worin auch 1-3 H-Atome durch F ersetzt sein können,
bedeuten,
sowie ihrer pharmazeutisch verwendbaren Derivate, Solvate und Stereoisomeren, einschließlich deren Mischungen in allen Verhältnissen.

10. Verfahren nach einem oder mehreren der Ansprüche 1-9,
wobei die Umsetzung der Stufe a) in einem inerten Lösungsmittel oder -gemisch, in Gegenwart eines Alkali- oder Erdalkalimetall-hydroxids, -carbonats oder -bicarbonats erfolgt.

11. Verfahren nach einem oder mehreren der Ansprüche 1-10,
wobei die Umsetzung der Stufe a) in wässriger NaHCO₃-Lösung erfolgt.

12. Verfahren nach einem oder mehreren der Ansprüche 1-11,
wobei die Umsetzung der Stufe a) bei einer Temperatur zwischen 60° und 110° C erfolgt.

13. Verfahren nach einem oder mehreren der Ansprüche 1-12,
wobei in Stufe b) die Umsetzung in Gegenwart von Ethyl-2-ethoxy-1,2-dihydrochinolin-1-carboxylat (EEDQ) erfolgt.

14. Verfahren nach einem oder mehreren der Ansprüche 1-13,
wobei die Umsetzung der Stufe b) bei einer Temperatur zwischen 10° und 70° C erfolgt.

15. Verfahren nach einem oder mehreren der Ansprüche 1-14,
wobei die Umsetzung der Stufe b) in Tetrahydrofuran erfolgt.

16. Verfahren nach einem oder mehreren der Ansprüche 1-15, zur Herstellung von Verbindungen der Formel la 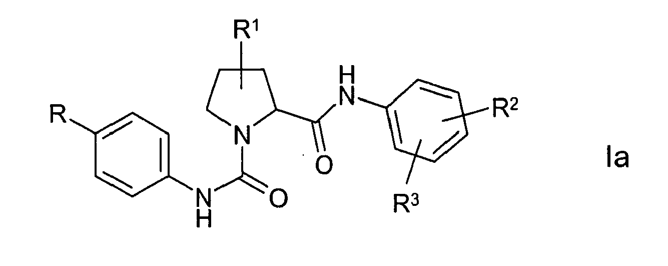 worin
R F oder Cl,
R¹ H oder OH,
R² H, F oder A,
R³ 3-Oxo-morpholin-4-yl,
A unverzweigtes oder verzweigtes Alkyl mit 1-6 C-Atomen, worin auch 1-3 H-Atome durch F ersetzt sein können,
bedeuten,
sowie ihrer pharmazeutisch verwendbaren Derivate, Solvate und Stereoisomeren, einschließlich deren Mischungen in allen Verhältnissen, **dadurch gekennzeichnet, daß** man
a) eine Verbindung der Formel II 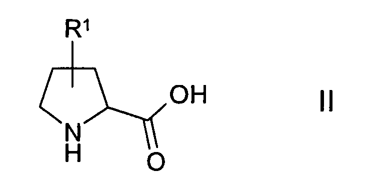 worin
R¹ H oder OH bedeutet,
mit einer Verbindung der Formel III 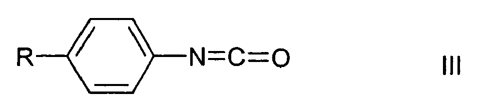 worin
R F oder CI bedeutet,
in wässriger Alkali- oder Erdalkalimetall-carbonat- oder -bicarbonat-Lösung, bei einer Temperatur zwischen 60° und 110° C,
zu einer Verbindung der Formel IV 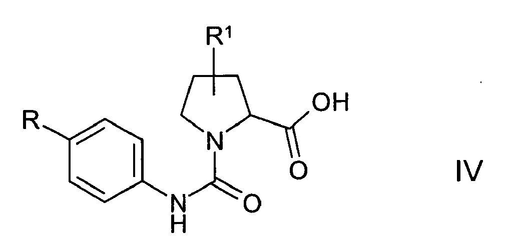 worin
R F oder Cl,
R¹ H oder OH
bedeuten,
umsetzt,
b) dann eine Verbindung der Formel IV mit einer Verbindung der Formel V 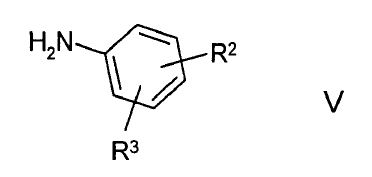 worin
R² H, F oder A,
R³ 3-Oxo-morpholin-4-yl,
A unverzweigtes oder verzweigtes Alkyl mit 1-6 C-Atomen, worin auch 1-3 H-Atome durch F ersetzt sein können,
bedeuten,
in Gegenwart eines Hilfsreagenzes unter Bildung eines gemischten Anhydrids, bei einer Temperatur zwischen 10° und 70° C,
zu einer Verbindung der Formel la umsetzt, und
c) diese gegebenenfalls in ihre pharmazeutisch verwendbaren Derivate und/oder Solvate überführt,
indem man eine Base oder Säure der Formel la in eines ihrer Salze umwandelt.

17. Verfahren nach einem oder mehreren der Ansprüche 1-16, zur Herstellung von Verbindungen ausgewählt aus der Gruppe
(2R,4R)-4-Hydroxy-pyrrolidin-1,2-dicarbonsäure-1-[(4-chlorphenyl)-amid]-2-{[4-(3-oxo-morpholin-4-yl)-phenyl]-amid},
(2R)-Pyrrolidin-1,2-dicarbonsäure-1-[(4-chlor-phenyl)-amid]-2-{[4-(3-oxo-morpholin-4-yl)-phenyl]-amid},
(2R)-Pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-chlorphenyl)-amid]-2-{[3-methyl-4-(3-oxo-morpholin-4-yl)-phenyl]-amid},
(2S,4R)-4-Hydroxy-pyrrolidin-1,2-dicarbonsäure-1-[(4-chlorphenyl)-amid]-2-{[4-(3-oxo-morpholin-4-yl)-phenyl]-amid},
(2R)-Pyrrolidin-1,2-dicarbonsäure-1-[(4-chlor-phenyl)-amid]-2-{[2-fluor-4-(3-oxo-morpholin-4-yl)-phenyl]-amid},
(2R,4S)-4-Hydroxy-pyrrolidin-1,2-dicarbonsäure-1-[(4-chlorphenyl)-amid]-2-{[4-(3-oxo-morpholin-4-yl)-phenyl]-amid},
(2R)-Pyrrolidin-1,2-dicarbonsäure-1-[(4-chlor-phenyl)-amid]-2-{[3-fluor-4-(3-oxo-morpholin-4-yl)-phenyl]-amid},
(2R)-Pyrrolidin-1,2-dicarbonsäure-1-[(4-chlor-phenyl)-amid]-2-{[3-trifluormethyl-4-(3-oxo-morpholin-4-yl)-phenyl]-amid},
(2R,4R)-4-Hydroxy-pyrrolidin-1,2-dicarbonsäure-1-[(4-chlorphenyl)-amid]-2-{[2-fluor-4-(3-oxo-morpholin-4-yl)-phenyl]-amid},
(2R,4S)-4-Azido-pyrrolidin-1,2-dicarbonsäure-1-[(4-chlor-phenyl)-amid]-2-{[4-(3-oxo-morpholin-4-yl)-phenyl]-amid},
(2R,4S)-4-Amino-pyrrolidin-1,2-dicarbonsäure-1-[(4-chlorphenyl)-amid]-2-{[4-(3-oxo-morpholin-4-yl)-phenyl]-amid},
(2R,4R)-4-Methoxy-pyrrolidin-1,2-dicarbonsäure-1-[(4-chlorphenyl)-amid]-2-{[4-(3-oxo-morpholin-4-yl)-phenyl]-amid},
(2 R,4R)-4-Acetoxy-pyrrolidin-1,2-d icarbonsäure-1-[(4-chlorphenyl)-amid]-2-{[4-(3-oxo-morpholin-4-yl)-phenyl]-amid},
(2R)-4-Oxo-pyrrolidin-1,2-dicarbonsäure-1-[(4-chlor-phenyl)-amid]-2-{[4-(3-oxo-morpholin-4-yl)-phenyl]-amid},
(2S)-Pyrrolidin-1,2-dicarbonsäure-1-[(4-chlor-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-chlorphenyl)-amid]-2-{[3-fluor-4-(3-oxo-morpholin-4-yl)-phenyl]-amid},
(2S,4S)-4-Hydroxy-pyrrolidin-1,2-dicarbonsäure-1-[(4-chlorphenyl)-amid]-2-{[4-(3-oxo-morpholin-4-yl)-phenyl]-amid},
(2 R,4R)-4-Allyloxy-pyrrolidin-1,2-dicarbonsäure-1-[(4-chlorphenyl)-amid]-2-{[4-(3-oxo-morpholin-4-yl)-phenyl]-amid},
(2R,4R)-4-(Prop-2-in-yloxy)-pyrrolidin-1,2-dicarbonsäure-1-[(4-chlor-phenyl)-amid]-2-{[4-(3-oxo-morpholin-4-yl)-phenyl]-amid},
sowie ihrer pharmazeutisch verwendbaren Derivate, Solvate und Stereoisomeren, einschließlich deren Mischungen in allen Verhältnissen.

18. Verbindungen der Formel IV 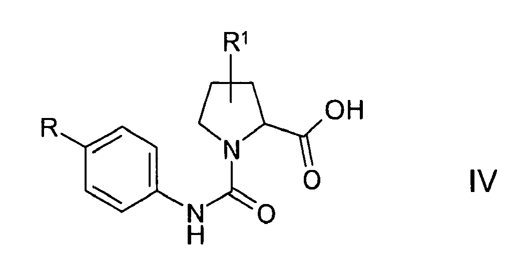 worin
R Hal oder C≡CH,
R¹ H, =O, Hal, A, OH, OA, A-COO-, A-CONH-, A-CONA-, N₃, NH₂, NO₂, CN, COOH, COOA, CONHA, CONH₂, CON(A)₂, O-Allyl, O-Propargyl, O-Benzyl, =N-OH oder =N-OA,
A unverzweigtes, verzweigtes oder cylisches Alkyl mit 1-10 C-Atomen, worin auch 1-7 H-Atome durch F ersetzt sein können,
Hal F, Cl, Br oder I,
bedeuten,
sowie ihre pharmazeutisch verwendbaren Derivate, Solvate und Stereoisomere, einschließlich deren Mischungen in allen Verhältnissen.

19. Verbindungen nach Anspruch 18,
worin
R F oder Cl,
R¹ H, =O, OH, OA, A-COO-, N₃, NH₂, O-Allyl oder O-Propargyl
bedeuten,
sowie ihre pharmazeutisch verwendbaren Derivate, Solvate und Stereoisomere, einschließlich deren Mischungen in allen Verhältnissen.

20. Verbindungen nach Anspruch 18 oder 19,
worin
R F oder Cl,
R¹ H oder OH
bedeuten,
sowie ihre pharmazeutisch verwendbaren Derivate, Solvate und Stereoisomere, einschließlich deren Mischungen in allen Verhältnissen.
